(19) Europäisches Patentamt
European Patent Office
Office européen des brevets

(11) Publication number: 0 086 430 B1

(12) # EUROPEAN PATENT SPECIFICATION

(45) Date of publication of patent specification: **06.11.85**

(51) Int. Cl.$^4$: **C 07 C 50/36**

(21) Application number: **83101145.7**

(22) Date of filing: **17.09.81**

(60) Publication number of the earlier application in accordance with Art. 76 EPC: **0 049 403**

(54) **Anthracyclinone derivatives and process for their production.**

(30) Priority: **22.09.80 JP 130645/80**

(43) Date of publication of application: **24.08.83 Bulletin 83/34**

(45) Publication of the grant of the patent: **06.11.85 Bulletin 85/45**

(84) Designated Contracting States: **DE FR GB IT SE**

(56) References cited:
**GB-A-2 036 021**

**TETRAHEDRON LETTERS, vol. 21, no. 27, 1980, pages 2661-2664, Pergamon Press, Oxford (GB); A.V. RAMA RAO et al.: "A simple synthesis of (+) 4-demethoxydaunomycinone"**

**THE JOURNAL OF ANTIBIOTICS, vol. XXXIII, no. 12, December 1980, pages 1581-1585; H. UMEZAWA: "Synthesis of 4-demethoxy-11-deoxy-analogs of daunomycin and adriamycin"**

(73) Proprietor: **ZAIDAN HOJIN BISEIBUTSU KAGAKU KENKYU KAI**
**14-23, Kamiosaki 3-chome**
**Shinagawa-ku Tokyo (JP)**

(72) Inventor: **Umezawa, Hamao**
**23, Toyotama Kita 4-chome**
**Nerima-ku Tokyo (JP)**
Inventor: **Takeuchi, Tomio**
**5-1-11, Higashigotanda**
**Shinagawa-ku Tokyo (JP)**
Inventor: **Naganawa, Hiroshi**
**3-17, Denenchofu Hon-cho**
**Ohta-ku Tokyo (JP)**
Inventor: **Tatsuta, Kuniaki**
**26-7, Matsunoki 2-chome**
**Suginami-ku Tokyo (JP)**

(74) Representative: **Kraus, Walter, Dr. et al**
**Patentanwälte Kraus Weisert & Partner Thomas-Wimmer-Ring 15**
**D-8000 München 22 (DE)**

**EP 0 086 430 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to novel anthracyclinone derivatives.

Daunomycin (see US—A—3,616,242) and adriamycin (see US—A—3,590,028) obtained from fermentation broths of microorganisms of genus Actinomyces have been known as anthracycline-type antibiotics. These compounds have a broad antitumor spectrum against experimental tumors, and are widely used clinically as chemotherapeutic antitumor agents. Daunomycin and adriamycin show considerably strong antitumor actions, but these actions are never satisfactory. Numerous attempts have been made to create various analogous compounds having stronger antitumor actions by various means such as a fermentation method, a semisynthetic method, a totally synthetic method or a microorganism converting method. Some specific methods have already been proposed [see, for example, F. Arcamone, Topics in antibiotic Chemistry, Vol. 2, pages 102 to 279, Ellis Horwood Limited; U.S. Patent No. 3,988,315 (aclacinomycins A and B)].

The object of the present invention is to provide important intermediates for the synthesis of anthracyclinone-type compounds which are useful as medicines and which are analoguous to Daunomycin and Adriamycin and which have higher activity.

This object is achieved by the present invention.

The invention relates to anthracyclinone derivatives of the following formula

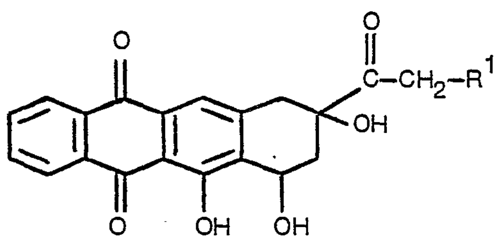

(I—b)

wherein $R^1$ represents a hydrogen atom, hydroxyl group or lower alkanyl-oxy group.

The invention includes 4-demethoxy-11-deoxydaunomycinone ($R^1$=H), or 4-demethoxy-11-deoxyadriamycinone or its 14-lower alkanoyl derivatives ($R^1$=H or lower alkanoyloxy group). These compounds are useful as important intermediates for the synthesis of anthracyclinone-type compounds which are useful as medicines.

In the present application, the term "lower" used to qualify groups or compounds means that the groups or compounds so qualified contain not more than 6, preferably not more than 4, carbon atoms.

The "lower alkanoyloxy group" represented by $R^1$ in formula (I) denotes a residue of an alkanoic acid whose alkyl moiety is linear or branched and contains 1 to 5, preferably 1 to 3, carbon atoms. Examples are acetyl, propionyl, n-butyryl, isobutyryl, n-valeryl and isovaleryl. The acetyl group is especially preferred as the lower alkanoyloxy group.

The compound of formula (I) provided by the present invention can be totally synthesized, for example, by a synthetic route shown by the following scheme.

REACTION SCHEME

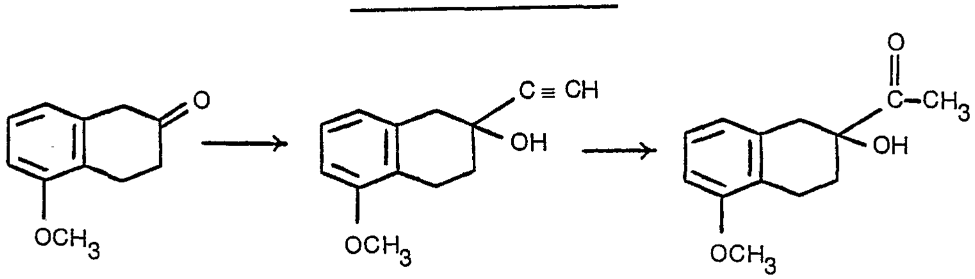

(1) (2) (3)

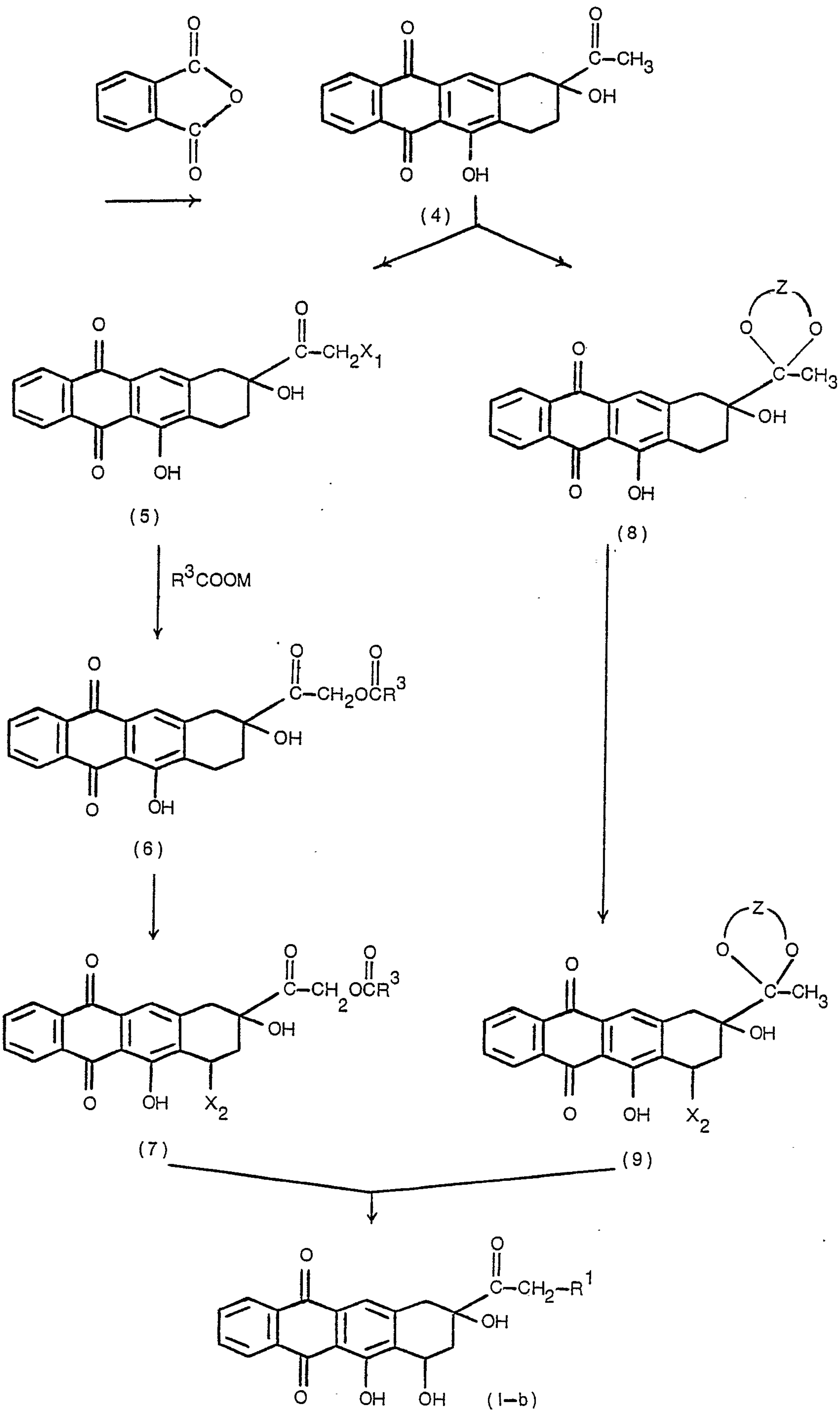


(4)

(5)

(8)

(6)

(7)

(9)

(I–b)

In the above reaction scheme, $R^1$ is as defined hereinabove; $R^3$ represents a lower alkyl group; $X_1$ and $X_2$ represent a halogen atom, especially a bromine atom; Z represents a ketal residue; and M represents an alkali metal atom, an alkaline earth metal atom or an ammonium group.

The unit reaction in each of the steps in the above reaction scheme is known *per se*, and can be practiced by known methods. The reactions in the individual steps are briefly described below.

(1) → (2)

In this step, 5-methoxy-2-tetralone of formula (1) is reacted with a Grignard reagent of the formula $(CH{\equiv}C)MgX_3$ (wherein $X_3$ represents a halogen atom, especially a bromine atom) to yield a tetraline derivative of formula (2). This reaction can be carried out by utilizing a known Grignard reaction, for example as shown in step A in Example 1 to be given hereinbelow.

(2) → (3)

In this step, hydrolysis of the tetraline derivative of formula (2) gives 2-acetyl-2-hydroxy-5-methoxy-1,2,3,4-tetrahydronaphthalene. The hydrolysis can be carried out, for example, by treating the compound of formula (2) with sulfuric acid in the presence of mercuric oxide usually at room temperature.

The resulting compound of formula (3) can be isolated from the reaction mixture by an ordinary purifying method, for example by chromatography.

(3) → (4)

In this step, the compound of formula (3) prepared as above is reacted with phthalic anhydride to give 4-demethoxy-7,11-dideoxydaunomycinone of formula (4). The above reaction can be performed in accordance with the Friedel-Crafts reaction. For exmple, this reaction can be carried out in the presence of a Lewis acid such as aluminum chloride, titanium tetrachloride or zinc chloride at a temperature of 50 to 250°C, especially 150 to 200°C, and can be completed in 1 to 30 minutes, preferably in 5 to 10 minutes. The phthalic anhydride is used in an amount of at least 1 mole, preferably 1.5 to 2 moles, per mole of the compound of formula (3). The Lewis acid is used in an amount of at least 8 equivalents, preferably 12 to 20 equivalents, per mole of the compound of formula (3). Preferably, the Friedel-Crafts reaction is carried out in the absence of a solvent at a relatively high temperature at which the starting materials substantially melt, usually at at least about 150°C, preferably 170 to 190°C. This effectively prevents the occurrence of the side-reaction mentioned below.

If the Friedel-Crafts reaction is carried out in the presence of an ordinary organic solvent or at a relatively low temperature (lower than about 140°C), a compound of the following formula

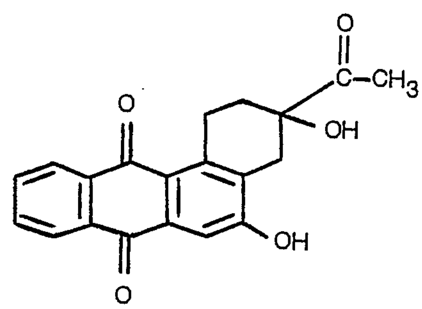

forms as a by-product in addition to the desired compound of formula (4), and decreases the yield of the compound of formula (4). It has been found that if the reaction is carried out in the absence of a solvent at a relatively high temperature, the compound of formula (4) can be obtained selectively in a high yield without the formation of such a by-product.

(4) → (5) → (6)

The reaction in this step can be carried out in a manner known *per se*, for example by the method described in U.S. Patent No. 3,803,124. Or the halogenation of the compound of formula (4) can be carried out by using an N-haloacid imide such as N-bromosuccinimide or N-chlorosuccinimide, especially the former, resulting in selective halogenation of the 14-position of the compound of formula (4). The halogenation with the N-haloacid imide can be carried out usually at a temperature of 0 to 50°C for about 2 to about 10 hours. The amount of the N-haloacid imide used is not critical. Generally, it is suitably used in an amount of at least 2 moles, preferably 8 to 16 moles, per mole of the compound of formula (4).

The acylation of the compound of formula (5) with a salt of an organic carboxylic acid having the formula $R^3COOM$ can be carried out at 0 to 80°C for about 5 to about 20 hours usually in an inert solvent, such as a ketone (e.g., acetone or methyl isobutyl ketone), or an ether (e.g., tetrahydrofuran or dioxane)

either alone or as a mixture. The salt of an organic carboxylic acid can be used generally in an amount of 1.5 moles, preferably 2 to 4 moles, per mole of the compound of formula (5).

Examples of the salt of an organic carboxylic acid include sodium acetate, potassium acetate, ammonium acetate, sodium propionate, potassium propionate, ammonium propionate, sodium valerate and ammonium valerate.

(4) → (8)

The reaction in this stage is ketalization of the carbonyl group at the 13-position of the compound of formula (4), and can be carried out in a manner known *per se* using a carbonyl protecting reagent (ketalizing agent) known *per se*. For example, it can be performed by the action of a ketalizing agent of the formula HO—Z—OH on the compound of formula (4) in a suitable solvent in the presence of an acid catalyst, for example an aromatic sulfonic acid such as p-toluenesulfonic acid or benzenesulfonic acid. The reaction temperature is not critical. Generally, the suitable temperature is from room temperature to the refluxing temperature of the reaction mixture, preferably from about 80°C to the refluxing temperature of the reaction mixture. The ketalizing agent is used in an amount of at least 1.5 moles, preferably from 5 to 15 moles, per mole of the compound of formula (4).

Specific examples of the ketal residue Z are

$-CH_2CH_2-$, 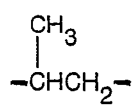, 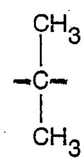 and 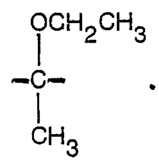.

Hence, specific examples of the ketalizing agent include ethylene glycol, propylene glycol, 1,1-dimethoxypropane and triethoxymethane.

(6) → (7) and (8) → (9)

These steps involve halogenation of the 7-position of the compound of formula (6) or (8). The halogenation can be carried out by using known halogenating agents such as bromine, chlorine, N-bromosuccinimide and N-chlorosuccinimide in a customary manner. Bromination is especially preferred.

In a preferred embodiment, a solution containing bromine is added to the above compound in an aqueous medium in the presence of a free radical generator such as 2,2-azobisisobutyronitrile (to be abbreviated AIBN), and the reaction is carried out generally at 0 to 60°C, preferably at room temperature for 1 to 10 hours. The amount of the bromine solution is generally at least 1.5 moles, preferably 2 to 3 moles as bromine, per mole of the compound (6) or (8).

(7) or (9) → (I-b)

The reaction in this step is hydrolysis. Since the compound of formula (7) or (9) is unstable, when the above halogenation step is carried out in the presence of water, the hydrolysis of the compound (7) or (9) readily occurs to give the corresponding compound of formula (I-b). The above hydrolysis is carried out by a conventional method using an aqueous alkali solution under mild conditions, for example at a relatively low temperature of from room temperature to about 50°C using a weak alkali such as sodium hydrogen carbonate.

When the hydrolysis of the compound of formula (7) is performed under relatively strong hydrolyzing conditions, for example, by treating this compound with 10% potassium carbonate ($K_2CO_3$) at room temperature for 1 hour, a compound of formula (I-b) wherein $R^1$ is a hydroxyl group is formed.

The compound of formula (I-b) is generally obtained as a mixture of four stereoisomers having configurations (7S, 9S), (7R, 9R), (7R, 9S) and (7S, 9R).

A racemic mixture (7S, 9S; 7R, 9R) and a racemic mixture (7R, 9S; 7S, 9R) can be easily separated from the stereoisomeric mixture of the compounds of formula (I-b) in a customary manner, for example by a chromatographic technique.

It is especially preferred that the desired compound of formula (I-b) in this invention has a configuration (7S, 9S). Accordingly, the separated racemic mixture (7R, 9S; 7S, 9R) can be epimerized to a racemic mixture (7S, 9S, 7R, 9R). As a result, the yield of the latter can be increased. The epimerization can be carried out, for example, by contacting the racemic mixture of the compound of formula (I-b) with an inorganic acid such as hydrochloric acid or perchloric acid in a water-soluble organic solvent such as acetone, tetrahydrofuran or dioxane at a temperature of from 10°C to the boiling point of the solvent.

The compounds of the present invention are useful as intermediates for the production of axcellent antitumor agents of formula (I-a) according to the following reaction scheme:

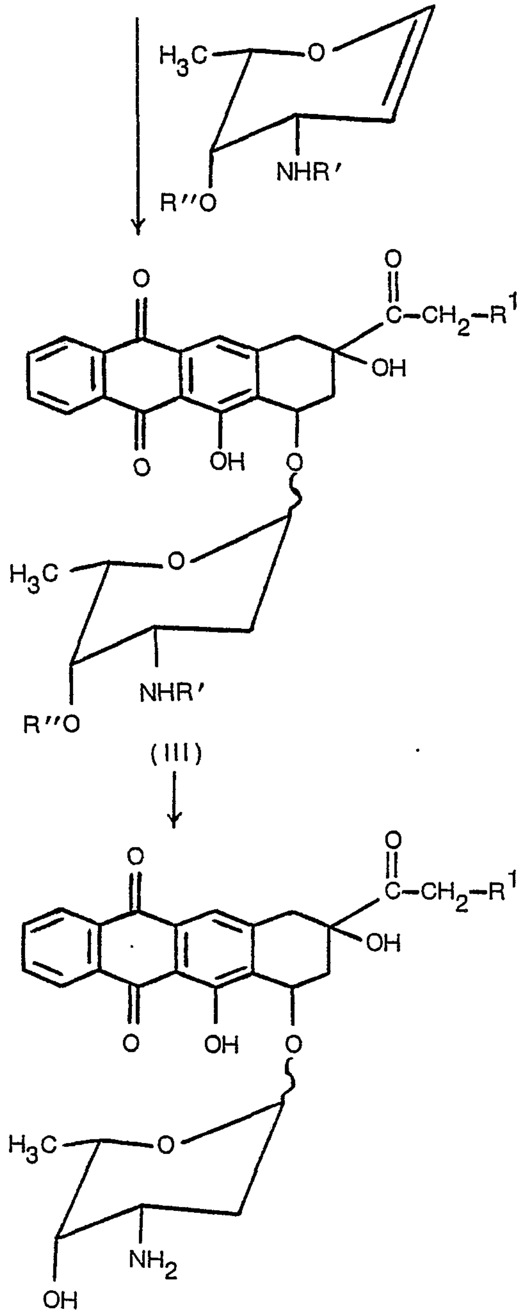

(III)

(I–a)

In the above reaction scheme, $R^1$ is defined as above and R′ and R″, resp., represent an amino-protecting group and a hydroxyl-protecting group which can be easily eliminated by hydrolysis.

The production process of these anti-tumor agents comprises the following steps:

(I-b) → (III)

In this step, the compound of formula (I-b) is reacted with a glycal of the formula

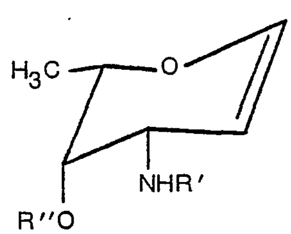

(10)

derived from daunosamine to give a glycoside of formula (III).

The amino-protecting group R′ in formula (10) may be those which can be easily eliminated by hydrolysis. Specific examples include lower alkanoyl groups such as acetyl, propionyl and trifluoroacetyl, aromatic carbonyl groups such as benzoyl and p-nitrobenzoyl, aralkyloxycarbonyl groups such as benzyloxycarbonyl, and alkyloxycarbonyl groups such as t-butoxycarbonyl. The hydroxy-protecting groups R″ which can be easily eliminated by hydrolysis may be similar groups to those cited above for the amino-protecting group R′.

The glycosidation reaction can be carried out by a method known *per se* using an acid catalyst. Usually, it is carried out in an anhydrous organic solvent, for example benzene, toluene, tetrahydrofuran or dioxane, preferably in an aromatic hydrocarbon such as benzene or toluene in the presence of an acid catalyst, for example an aromatic sulfonic acid such as p-toluenesulfonic acid or benzenesulfonic acid or an alkylsulfonic acid such as methanesulfonic acid or butanesulfonic acid. The reaction temperature is advantageously 0 to 80°C, preferably 20 to 40°C. It is advantageous to use the compound of formula (10) in an amount of at least 1.5 moles, preferably 2 to 4 moles, per mole of the compound of formula (I-b).

The glycal of formula (10) can be produced by treating a daunosamine derivative having the 3-amino group and the 4-hydroxyl group protected with the above-exemplfied groups, with a sulfonylating agent such as p-toluenesulfonyl chloride or benzenesulfonyl chloride in the presence of a base such as pyridine, dimethylaniline, morpholine or triethylamine in an organic solvent or in the absence of a solvent (see Japanese Patent Publication No. 27346/1979).

The glycoside of formula (III) according to the above glycoside-forming reaction is obtained usually as a mixture of an isomer whose amino sugar residue has a 1′α linkage (to be referred to as a 1′α isomer) and an isomer whose amino sugar residue has a 1′β-linkage (to be referred to as a 1′β isomer), and generally the proportion of the 1′α isomer is major.

Accordingly, when the racemic mixture (7S, 9S; 7R, 9R) of the compound of formula (I-b-1) is used as a starting material, the glycoside of formula (III) is a mixture of four steric isomers (7S, 9S, 1′α), (7S, 9S, 1′β), (7R, 9R, 1′α) and (7R, 9R, 1′β). These isomers can be separated individually by, for example, chromatography on silica gel or the like.

Isolation of these stereoisomers may be performed after the deprotecting reaction to be described hereinbelow.

(III) → (I-a)

This step is the elimination of the protective groups of the compound of formula (III). This deprotecting reaction is carried out by a known hydrolyzing method. For example, it can be effected by alkaline hydrolysis at a relatively low temperature of from about 0°C to room temperature in the presence of an alkali such as sodium carbonate, potassium carbonate. This hydrolysis results in the elimination of the protective groups R′ and R″ from the compound of formula (III). When $R^{12}$ is a lower alkanoyloxy group, the lower alkanoyl group is also eliminated depending upon the conditions of hydrolysis to give a compound of formula (I-a) in which $R^1$ is OH.

Thus, the anthracycline derivative of formula (I-a) can be obtained in a good yield.

The aglycone moiety shown in the present application is drawn in a planar structure, but is meant to include all configurations (7S, 9S), (7R, 9R), (7S, 9R) and (7R, 9S).

The compound of formula (III) produced by the above process in which $R^1$ represents a hydrogen atom can be converted to a compound of formula (III) in which $R^1$ is a lower alkanoyloxy group by subjecting it to the steps (4), (5) and (6) in this sequence.

The compound of formula (I-a) obtained as above can be converted to an acid addition salt by treating it with an inorganic or organic acid of the types exemplified hereinabove by methods known *per se*.

The compounds of formula (I-a) have excellent antitumor activity, and those having the configuration (7S, 9S) which is the same as daunomycin or adriamycin produced by a fermentation method. The glycoside desirably has a 1′α-linkage.

Example 1

Production of 4-demethoxy-11-deoxydaunomycinone:—

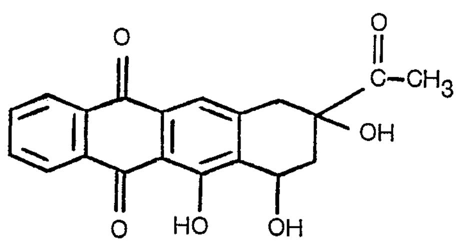

*Step A*

2-Ethinyl-2-hydroxy-5-methoxy-1,2,3,4-tetrahydronaphthalene:—

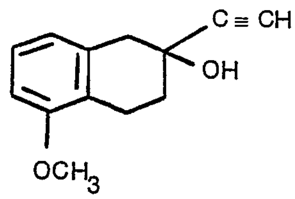

(2)

While acetylene was blown into 80 ml of anhydrous tetrahydrofuran; 36 ml of a 2M ether solution of ethylmagnesium bromide was added dropwise.

To the resulting solution was added 2.88 g of 5-methoxy-2-tetralone. After the reaction, the reaction mixture was poured into 500 ml of a saturated aqueous solution of ammonium chloride, and the mixture was extracted with 200 ml of carbon tetrachloride thrice. The extracts were washed with water, dried over sodium sulfate, and concentrated to dryness. The resulting brown oily product was chromatographed on a column of silica gel using benzene/ethyl acetate (25/1) as an eluent to give 1.6 g of the desired product as a brown oil.

NMR δ: 60 MHz, $CDCl_3$

1.9—2.3 (m) $—CH_2—$ at 3-position
2.40 (s) —C≡CH at 2-position
2.42 (s) —OH at 2 position
2.7—3.1 (m) $—CH_2—$ at 4-position
3.05—3.2 (m) $—CH_2—$ at 1-position
3.82 (s) $—OCH_3$ at 5-position
6.6—7.35 (m) H at 6-, 7- and -8-positions

*Step B*

2-Acetyl-2-hydroxy-5-methoxy-1,2,3,4-tetrahydronaphthalene:—

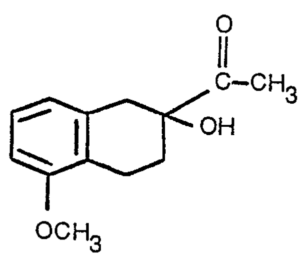

(3)

The compound of formula (2) was dissolved in 10 ml of carbon tetrachloride, and 10 ml of 1.5 N sulfuric acid and 200 mg of mercuric oxide were added. They were reacted at room temperature for 27 hours. Water (40 ml) was added, and the reaction mixture was extracted with 50 ml of carbon tetrachloride four times. The extracts were washed with water, dried over sodium sulfate, and concentrated to dryness. The resulting oily product was chromatographed on a column of silica gel using benzene/ethyl acetate (20/1) as an eluent to give 840 mg of the desired product as colorless needles.

Melting point: 63—63.5°C

NMR δ: 60 MHz, $CDCl_3$

1.8—2.1 (m) $—CH_2—$ at 3-position
2.28 (s) $COCH_3$ at 2-position
2.5—3.5 (m) $—CH_2—$ at 1- and 4-positions
3.45 (s) OH at 2-position
3.83 (s) $OCH_3$ at 5-position
6.6—7.3 (m) H at 6-, 8- and 8-positions

*Step C*

4-Demethoxy-7,11-dideoxydaunomycinone:—

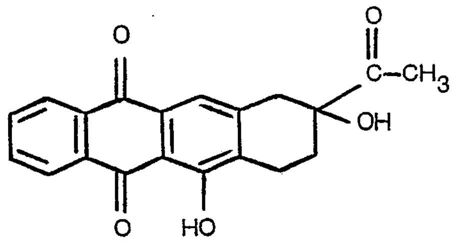

(4)

230 mg of the compound (3) obtained in step B, 230 mg of phthalic anhydride, 460 mg of sodium chloride and 2.3 g of aluminum chloride were well mixed, and melted by heating them to 180°C. In 1 to 2 minutes, the mixture became homogeneous, but was reacted further for 5 minutes.

The reaction mixture was treated with a saturated aqueous solution of oxalic acid, and extracted with 30 ml of chloroform five times. The extracts were combined, washed with water, dried over sodium sulfate, and concentrated to dryness. The product was chromatographed on a column of silica gel using benzene/ethyl acetate (20/1) as an eluent to give 110 mg of the desired product as a yellow solid.

Melting point: 208—214°C (decomp.)

NMR δ: 60 MHz, DMSO

1,7—2.2 (m) $—CH_2—$ at 8-position
2.30 (s) $—COCH_3$ at 9-position
2.6—3.1 (m) $—CH_2—$ at 7- and 10-positions
5.60 (s) —OH at 9-position
7.38 (s) —H at 11-position
7.8—8.4 (m) —H at 1-, 2-, 3- and 4-positions
12.80 (s) —OH at 6-position

*Step D*

Ketal compound of the compound (4):—

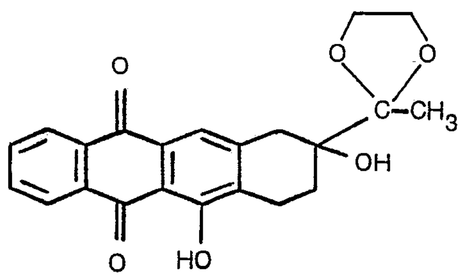

(8—a)

100 mg of the compound (4) was dissolved in 15 ml of benzene, and 0.3 ml of ethylene glycol and a catalytic amount of p-toluenesulfonic acid were added. They were reacted for 3 hours under reflux. The reaction mixture was poured into a 0.02 N aqueous solution of sodium hydrogen carbonate, and extracted with 40 ml of ethyl acetate three times. The extracts were dried over sodium sulfate, and concentrated to dryness. The above reaction proceeded quantitatively.

*Step E*

4-Demethoxy-11-deoxydaunomycinone:—

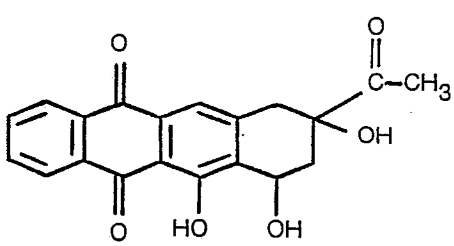

The ketal compound (8-a) obtained in step D was dissolved in chloroform, and 12.6 ml of 0.8% (W/V) bromine in carbon tetrachloride, 21 ml of water and 120 mg of azobisisobutyronitrile were added, and they

were reacted at room temperature for 3.5 hours. After the reaction, 4 ml of the above bromine solution was further added, and the reaction was carried out for 2 hours. After the reaction, bromine, carbon tetrachloride and chloroform were distilled off from the reaction mixture. The residue was dissolved in 120 ml of acetone, and 21 ml of 8N hydrochloric acid was added. The reaction was carried out at room temperature for 18 hours [deprotection of the ketal, and the epimerization of (7S, 9R; 7R, 9S) to (7S, 9S; 7R, 9R)]. After the reaction, acetone was distilled off from the reaction mixture, and the residue was extracted with 25 ml of chloroform twice. The extracts were washed with water, dried over sodium sulfate, and concentrated to dryness.

The product was treated with a crosslinked dextran gel (for example, Sephadex LH—20, a tradename for a product of Pharmacia Fine Chemicals) using acetone, and then the acetone was distilled off to give the desired product [a mixture of a racemic mixture (7S, 9R; 7R, 9S) and a racemic mixture (7S, 9S; 7R, 9R)]. The above stereoisomeric mixture was chromatographed on a column of silica gel using benzene/ethyl acetate (16/1), benzene/ethyl acetate (12/1), benzene/ethyl acetate (8/1) and then benzene/ethyl acetate (4/1) to give 41 mg of a stereoisomer (7S, 9S; 7R, 9R) of the above compound as a yellow solid and 21 mg of a stereoisomer (7S, 9R; 7R, 9S) of the above compound as a yellow solid.

*Step E'*

Epimerization of 4-demethoxy-11-deoxydaunomycinone (7S, 9R; 7R, 9S) to its isomer (7S, 9S; 7R, 9R):—

24 mg of the above (7S, 9R; 7R, 9S) isomer was dissolved in 15 ml of acetone, and 1.5 ml of 60% perchloric acid was added. The reaction was carried out at room temperature for 3 hours. The reaction mixture was worked up and purified in the same way as in step E to give 12 mg of 4-demethoxy-11-deoxydaunomycinone (7S, 9S; 7R, 9R).

Melting point: 199—207°C (decomp.)

NMR δ: 60 MHz, $CDCl_3$

2.0—2.5 (m) $—CH_2—$ at 8-position
2.44 (s) $—\overset{\overset{O}{\|}}{C}—CH_3$ at 9-position
3.14 (AB) $—CH_2—$ at 10-position
3.65 —OH at 7-position
4.60 (s) —OH at 9-position
5.36 (m) —H at 7-position
7.60 (s) —H at 11-position
7.7—7.9 (m) —H at 2- and 3-positions
8.2—8.4 (m) —H at 1- and 4-positions
13.23 (s) —OH at 6-position

Example 2

Production of 14-O-acetyl-4-demethoxy-11-deoxyadriamycinone:—

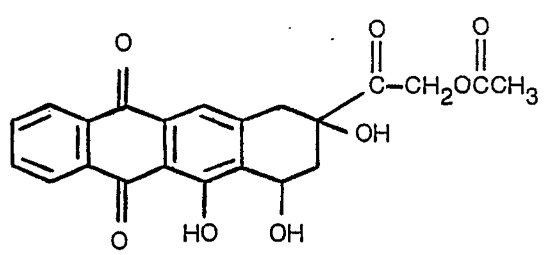

*Step A*

14-O-Acetyl-4-demethoxy-7,11-dideoxyadriamycinone:—

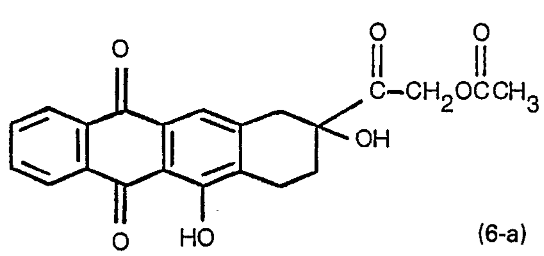

(6-a)

110 mg of the compound (4) obtained in step C of Example 1 was dissolved in 12 ml of tetrahydrofuran, and 200 mg of NBS was added four times at 1 hour intervals. The reaction was carried out at room temperature for a total period of 6 hours. The reaction mixture was poured into 200 ml of water, and extracted with 100 ml of carbon tetrachloride two times. The extracts were dried over sodium sulfate, and concentrated to dryness under reduced pressure. The product, without isolation and purification, was dissolved in 40 ml of acetone, and 200 mg of potassium acetate was added. The reaction was carried out at room temperature for 8 hours. After the reaction, the reaction mixture was poured into 50 ml of water, extracted with 26 ml of chloroform four times, dried over sodium sulfate, and then concentrated to dryness.

The product was chromatographed on a column of silica gel using benzene/ethyl acetate (40/1), benzene/ethyl acetate (30/1), benzene/ethyl acetate (20/1), and then benzene/ethyl acetate (15/1) as an eluent to give 83 mg of the title compound.

Melting point: 206—209°C

NMR δ: 100 MHz, $CDCl_3$

2.0—2.3 (m) —$Ch_2$— at 8-position

O
||
2.20 (s) —OC—$Ch_3$ at 9-position

2.79 (s) —OH at 9-position

2.8—3.5 (m) —$CH_2$— at 7- and 10-positions

O O
|| ||
5.11 (s) —$C\underline{H_2}$—O $CCH_3$

7.60 (s) —H at 11-position

7.75—7.9 (m) —H at 2- and 3-positions

8.2—8.4 (m) —H at 1- and 4-positions

13.03 (s) —OH at 6-position

*Step B*

14-O-Acetyl-4-demethoxy-11-deoxyadriamycinone:—

51 mg of the compound (6-a) was dissolved in 14 ml of chloroform, and 12 ml of water and 16 mg of azobisisobutyronitrile were added. To the solution was added 0.5 ml of 0.8% (W/V) bromine in carbon tetrachloride in seven portions at 1-hour intervals at room temperature, and the reaction was carried out for 9 hours. After the reaction, the unreacted bromine, carbon tetrachloride and chloroform were distilled off. The residue was dissolved in 40 ml of ethyl acetate, and 10 ml of a 0.1N aqueous solution of sodium hydrogen carbonate was added. The mixture was stirred at room temperature for a day and night. After the reaction, the reaction mixture was subjected to a separating procedure. The ethyl acetate layer was washed with water, dried over sodium sulfate, and concentrated to dryness. The dried product was chromatographed in the same way as in step A to give 34 mg of a racemic mixture (7S, 9S; 7R, 9R) of the title compound.

Melting point: 198—205°C (decomp.)

NMR δ: 100 MHz, $CDCl_3$

2.0—2.6 (m) —$CH_2$— at 8-position

O
||
2.19 (s) OC—$CH_3$ at 9-position

3.28 —OH at 7-position

3.22 (AB) —$CH_2$— at 10-position

4.69 (s) —OH at 9-position

O O
|| ||
5.25 (AB) —C—$\underline{CH_2}$—O $CCH_3$

5.42 (m) —H at 7-position

7.68 (s) —H at 11-position

7.75—7.9 (m) —H at 2- and 3-positions

8.25—8.4 (m) —H at 1- and 4-positions

13.40 (s) —OH at 6-position

IR ($cm^{-1}$)

3450, 2950, 1750, 1730, 1670, 1630, 1590, 1480, 1420, 1385, 1360, 1330, 1285, 1270, 1245, 1215, 1160, 1110, 1100, 1065, 1060, 1040, 1030, 1010, 980, 960, 930, 910, 870, 840, 830, 820, 795, 770, 745, 715.

**Claims**

1. An anthracyclinone derivative of the general formula

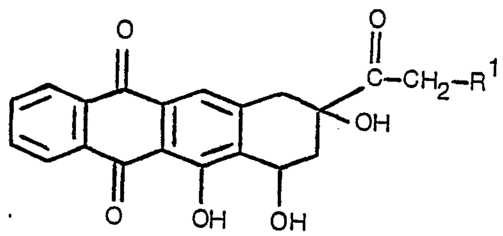

(I–b)

wherein $R^1$ represents a hydrogen atom, a hydroxyl group or a lower alkanoyloxy group.

2. A compound of claim 1 which is 4-demethoxy-11-deoxydaunomycinone.

3. A compound of claim 1 which is 4-demethoxy-11-deoxyadriamycinone or a 14-O-lower alkanoyl-4-demethoxy-11-deoxyadriamycinone.

4. A compound of any one of claims 1 to 3 wherein the anthracyclinone derivative is a racemic mixture (7S, 9S; 7R, 9R).

5. A process for producing a compound of the formula

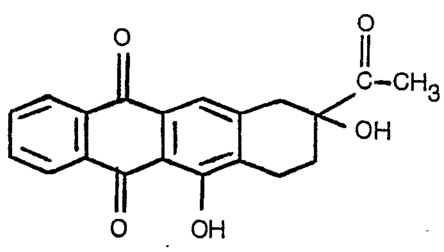

(4)

which comprises reacting a compound of the formula

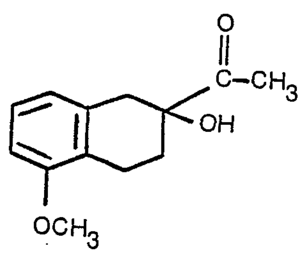

(3)

with phthalic anhydrid of the formula

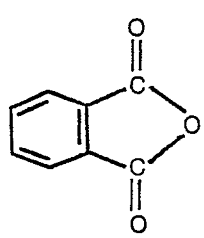

in the presence of a Lewis acid and in the absence of a solvent at a temperature of at least 150°C.

**Patentansprüche**

1. Anthracyclinonderivat der allgemeinen Formel

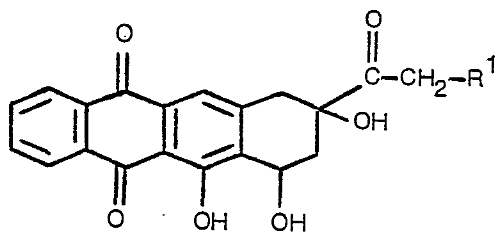

(I–b)

worin $R^1$ für ein Wasserstoffatom, eine Hydroxylgruppe oder eine Niedrigalkanoyloxygruppe steht.

2. Verbindung nach Anspruch 1, nämlich 4-Demethoxy-11-desoxy-daunomycinon.

3. Verbindung nach Anspruch 1, nämlich 4-Demethoxy-11-desoxyadriamycinon oder ein 14-O-Niedrigalkanoyl-4-demethoxy-11-desoxyadriamycinon.

4. Verbindung nach einem der Ansprüche 1 bis 3, worin das Anthracyclinonderivat ein racemisches Gemisch (7S, 9S; 7R, 9R) ist.

5. Verfahren zur Herstellung einer Verbindung der Formel

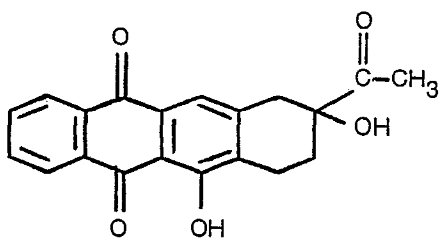

(4)

dadurch gekennzeichnet, daß man eine Verbindung der Formel

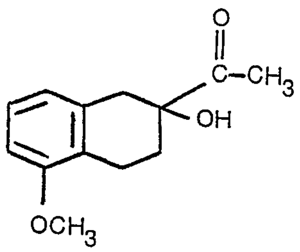

(3)

mit Phthalsäureanhydrid der Formel

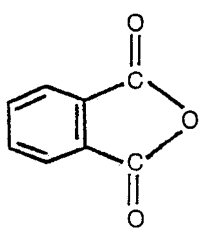

in Gegenwart einer Lewissäure und in Abwesenheit eines Lösungsmittels bei einer Temperatur von mindestens 150°C umsetzt.

**Revendications**

1. Les dérivés d'anthracyclinone de formule générale

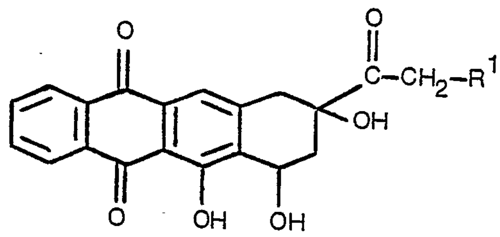

(I—b)

dans laquelle $R^1$ représente un atome d'hydrogène, un hdroxyle ou un groupe alcanoyloxy inférieur.

2. Composé selon la revendication 1 qui est la 4-déméthoxy-11-désoxydaunomycinone.

3. Composé selon la revendication 1 qui est la 4-déméthoxy-11-désoxyadriamycinone ou une 14-O-alcanoyl-4-déméthoxy-11-désoxyadriamycinone dont le groupe alcanoyle est un alcanoyle inférieur.

4. Composé selon l'une quelconque des revendications 1 à 3, caractérisé en ce que le dérivé d'anthracyclinone est un mélange racémique (7S, 9S; 7R, 9R).

5. Un procédé de production d'un composé de formule

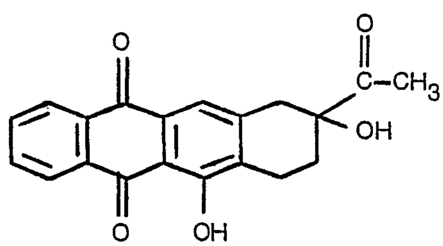

(4)

selon lequel on fait réagir un composé de formule

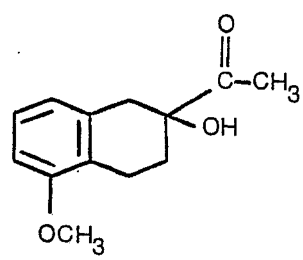

(3)

avec l'anhydride phtalique de formule

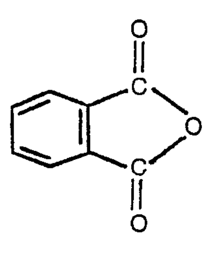

en présence d'un acide de Lewis, sans solvant et à une température d'au moins 150°C.